# EUROPÄISCHE PATENTSCHRIFT

(11) **EP 3 541 778 B1**
(45) Veröffentlichungstag und Bekanntmachung des Hinweises auf die Patenterteilung: **19.05.2021**
(21) Anmeldenummer: 17801651.5
(22) Anmeldetag: 10.11.2017
(51) Int. Cl.: C07C 68/00, C07C 69/96, C25B 3/00

(54) **ELEKTROCHEMISCHES VERFAHREN ZUR HERSTELLUNG VON ARYLALKYLCARBONATEN UND DIARYLCARBONATEN**
ELECTROCHEMICAL METHOD FOR THE PRODUCTION OF ARYLALKYLCARBONATES AND DIARYL CARBONATES
PROCÉDÉ ÉLECTROCHIMIQUE DESTINÉ À LA FABRICATION DE CARBONATES ARYLALKYL ET DE CARBONATES DIARYL

(30) Priorität: 15.11.2016 EP 16198768
(43) Veröffentlichungstag der Anmeldung: 25.09.2019
(73) Patentinhaber: Covestro Intellectual Property GmbH & Co. KG, 51373 Leverkusen (DE)
(72) Erfinder: TRIEU, Vinh, 50679 Köln (DE); WEBER, Rainer, 51519 Odenthal (DE); WALDVOGEL, Siegfried, R., 55435 Gau-Algesheim (DE); HEIJL, Jan, 9160 Lokeren (BE); GIESHOFF, Tile, 64285 Darmstadt (DE)
(74) Vertreter: Levpat
(86) Internationale Anmeldenummer: PCT/EP2017/078907
(87) Internationale Veröffentlichungsnummer: WO 2018/091370

(56) Entgegenhaltungen:
- EP-A1- 2 650 278
- SHINSUKE FUKUOKA ET AL: "A novel non-phosgene polycarbonate production process using by-product CO 2 as starting material", GREEN CHEMISTRY, Bd. 5, Nr. 5, 2003, Seiten 497-507, XP055331614, GB ISSN: 1463-9262, DOI: 10.1039/B304963A in der Anmeldung erwähnt
- TORU MURAYAMA ET AL: "Phosgene-Free Method for Diphenyl Carbonate Synthesis at the Pd 0 /Ketjenblack Anode", JOURNAL OF PHYSICAL CHEMISTRY C, Bd. 116, Nr. 19, 17. Mai 2012 (2012-05-17) , Seiten 10607-10616, XP055331612, US ISSN: 1932-7447, DOI: 10.1021/jp300809s in der Anmeldung erwähnt

## Beschreibung

Die Erfindung betrifft ein elektrochemisches Verfahren zur Herstellung von Arylalkylcarbonaten und Diarylcarbonaten

Die Erfindung geht aus von an sich bekannten elektrochemischen Verfahren zur Herstellung von Diarylcarbonaten.

Arylalkylcarbonate und Diarylcarbonate sind wichtige Vorstufen bei der Herstellung von Polycarbonaten.

Die Herstellung aromatischer Polycarbonate nach dem Schmelzumesterungsverfahren ist bekannt und beispielsweise beschrieben in "Schnell", Chemistry and Physics of Polycarbonates, Polymer Reviews, Vol. 9, Interscience Publishers, New York, London, Sydney 1964, in D.C. Prevorsek, B.T. Debona and Y. Kersten, Corporate Research Center, Allied Chemical Corporation, Moristown, New Jersey 07960, "Synthesis of Poly(ester)carbonate Copolymers" in Journal of Polymer Science, Polymer Chemistry Edition, Vol. 19, 75-90 (1980), in D. Freitag, U. Grigo, P.R. Müller, N. Nouvertne, BAYER AG, "Polycarbonates" in Encyclopedia of Polymer Science and Engineering, Vol. 11, Second Edition, 1988, Seiten 648-718 und schließlich in Dres. U. Grigo, K. Kircher und P.R. Müller "Polycarbonate" in Becker/Braun, Kunststoff-Handbuch, Band 3/1, Polycarbonate, Polyacetale, Polyester, Celluloseester, Carl Hanser Verlag München, Wien 1992, Seiten 117-299.

Die Herstellung der in dem Schmelzumesterungsverfahren für aromatische Polycarbonate eingesetzten Diarylcarbonate, z.B. durch den Phasengrenzflächenprozess, ist prinzipiell in der Literatur beschrieben, siehe z.B. in Chemistry and Physics of Polycarbonates, Polymer Reviews, H. Schnell, Vol. 9, John Wiley and Sons, Inc. (1964), S. 50/51. Die Darstellung des Diarylcarbonates erfolgt hierbei durch die Umsetzung von Phenol mit einem Carbonyldihalogenid (z.B. Phosgen), welches ausgehend von Kohlenmonoxid hergestellt wird.

Die Darstellung von Diarylcarbonaten kann ebenfalls über eine oxidative Carbonylierung erfolgen. Hierbei wird Phenol direkt mit Kohlenmonoxid als Carbonylierungsreagenz umgesetzt (Journal of Molecular Catalysis A: Chemical, 1999, 139, 109-119). Die oxidative Carbonylierung von Phenol ist auch als elektrochemische Variante bekannt, beschrieben in J. Phys. Chem. C, 2012, 116, 10607-10616, ACS Catal., 2013, 3, 389-392 und Res. Chem. Intermed., 2015, 41, 9497-9508. Bei der chemischen und auch der elektrochemischen oxidativen Carbonylierung zur Darstellung von Diarylcarbonaten ist die Notwendigkeit des Einsatzes von teuren Palladium-basierten Katalysatoren von großem Nachteil. Die oxidative Carbonylierung zur Darstellung von Diarylcarbonaten wird bisher nicht industriell angewandt.

Allgemein ist der Einsatz von Kohlenmonoxid bei den hier beschriebenen Verfahren ein Nachteil, da dieses überwiegend aus fossilen Rohstoffen hergestellt wird und daher kein nachhaltiger Rohstoff ist. Des Weiteren erfordert dessen Verwendung auch besondere Sicherheitsmaßnahmen.

Es wäre vorteilhafter, Kohlendioxid als alternativen Rohstoff für die Polymerproduktion zu verwenden. Auf diese Weise könnte man fossile Rohstoffe ersetzen und das Treibhausgas Kohlendioxid wieder in den Stoffkreislauf zurückführen, was allgemein auch als Schließen des Kohlenstoffkreislaufes bezeichnet wird. Insgesamt ließe sich so der Kohlendioxid-Fußabdruck verringern, was einen Beitrag zu weltweiten Klimaschutzzielen liefern würde. Kohlendioxid steht als Abfallprodukt in vielen chemischen Prozessen zur Verfügung und kann somit als nachhaltiger Rohstoff betrachtet werden. Ebenfalls vorteilhaft ist, dass Kohlendioxid als nicht brennbares Gas einfacher als Kohlenmonoxid zu handhaben ist.

In EP 2 650 278 ist ein Verfahren zur Herstellung von Diarylcarbonaten aus Dialkylcarbonaten beschrieben.

Für die Darstellung von Arylalkylcarbonaten und Diarylcarbonaten wird Kohlendioxid als Rohstoff bereits industriell eingesetzt, beschrieben in Green Chemistry, 2003, 5, 497-507. Ein großer Nachteil hierbei ist, dass für die darin beschriebene Synthese zahlreiche Syntheseschritte erforderlich sind: Ausgehend von Ethylenoxid, welches aus Ethylen hergestellt wird, erhält man zunächst nach Umsetzung mit Kohlendioxid Ethylencarbonat. Ethylencarbonat wird dann mit Methanol zu Dimethylcarbonat umgeestert. Anschließend erfolgt eine weitere Umesterung mit Phenol zum Arylalkylcarbonat, welches in einer letzten Reaktion mit Phenol zum Diarylcarbonat disproportioniert wird.

Daher ist es eine Aufgabe der Erfindung, eine Synthese mit Kohlendioxid als Rohstoff zu entwickeln, bei der weniger Syntheseschritte erforderlich sind. Die Schwierigkeit in der Nutzbarmachung von Kohlendioxid als Rohstoff ist, dass es reaktionsträge und energiearm ist. Es bedarf also einer Energiezufuhr, um Kohlendioxid in eine höherwertige Chemikalie umzuwandeln, die wieder in den Stoffkreislauf zurückgeführt werden kann. Damit auch der Kohlendioxid-Fußabdruck verringert werden kann, sollte die zusätzlich zugeführte Energie wiederum möglichst wenig Kohlendioxid-Emissionen verursachen. Dazu eignet sich beispielsweise elektrischer Strom, welcher in Form von Strom aus erneuerbarer Energieerzeugung mit geringen Kohlendioxid-Emissionen zur Verfügung steht. Es ist also besondere Aufgabe der Erfindung, ein elektrochemisches Verfahren zu entwickeln, bei der Strom direkt als Energiequelle eingesetzt werden kann, und bei dem Kohlendioxid als Vorprodukt eingesetzt werden kann.

Als nächstliegender Stand der Technik wird hier ein elektrochemisches Verfahren zur Herstellung von Diarylcarbonaten angesehen, bei dem Kohlenmonoxid als Carbonylierungsquelle eingesetzt wird. Gemäß J. Phys. Chem. C, 2012, 116, 10607-10616 erfolgt die elektrokatalytische Umsetzung an einem Palladiumkatalysator bestehend aus Nanopartikeln < 2 nm. Um die katalytische Aktivität des Palladiumkatalysators zu erhöhen hat die gleiche Arbeitsgruppe in nachfolgenden Arbeiten nur noch homogene Palladiumkomplexe verwendet, beschrieben in ACS Catal., 2013, 3, 389-392, Res. Chem. Intermed., 2015, 41, 9497-9508 und Catal. Sci. Technol., 2016, 6, 6002-6010.

Wie bereits oben beschrieben ist hier der Einsatz von teuren Palladium-basierten Katalysatoren von Nachteil, insbesondere wenn das Palladium als homogener Katalysator vorliegt und vom Produkt abgetrennt werden muss. Außerdem ist wie oben beschrieben die Nutzung von Kohlendioxid als Vorprodukt der Nutzung von Kohlenmonoxid vorzuziehen.

Die Aufgabe der vorliegenden Erfindung besteht darin, ein Verfahren zu entwickeln, bei dem unter anderem Kohlendioxid als Vorprodukt zur Herstellung von Arylalkylcarbonaten und Diarylcarbonaten eingesetzt wird. Hierbei sollten insbesondere nicht so viele Syntheseschritte erforderlich sein wie nach dem Stand der Technik.

Spezielle Aufgabe der Erfindung ist es, ein alternatives elektrochemisches Verfahren zur Herstellung von Arylalkylcarbonaten und Diarylcarbonaten bereitzustellen, bei dem unter anderem Kohlendioxid als Vorprodukt eingesetzt wird.

Die Aufgabe wird erfindungsgemäß dadurch gelöst, dass eine aromatische Verbindung mit einem Carbonat direkt anodisch gegebenenfalls in Anwesenheit eines aprotischen Lösungsmittels umgesetzt wird. Das eingesetzte Carbonat kann direkt aus Kohlendioxid in einer Vorstufe hergestellt werden.

Gegenstand der Erfindung ist ein Verfahren zur elektrochemischen Herstellung von Arylalkylcarbonaten und Diarylcarbonaten, dadurch gekennzeichnet, dass Verbindungen der Formel (1) 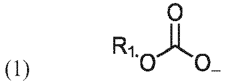 anodisch mit einer aromatischen Verbindung der Formel (2) 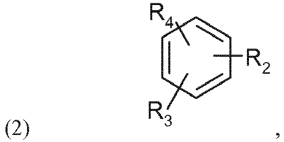 wobei der Rest R₁ einen Alkylrest bedeutet, bevorzugt einen Rest aus der Reihe: C₁- bis C₆- Alkyl, besonders bevorzugt Methyl-, Ethyl- oder tert-Butyl, oder Cycloalkyl-, bevorzugt Cyclohexyl-, oder einen Aryl-, Tert-butylphenyl-, Cumylphenyl- oder 4-Isopropylphenylrest, bevorzugt Naphthyl- oder Phenyl-, besonders bevorzugt einen Phenylrest bedeutet und die Reste R₂, R₃ und R₄ unabhängig voneinander Wasserstoff oder einen Alkylrest bedeuten, bevorzugt einen Rest aus der Reihe: C₁- bis C₆- Alkyl, iso-Propyl oder tert-Butyl oder Cycloalkyl, besonders bevorzugt Cyclohexyl bedeuten,
oder mit einer aromatischen Verbindung der Formel (3) 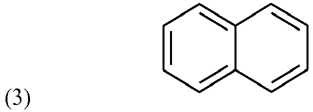 oder mit einer aromatischen Verbindung der Formel (4) 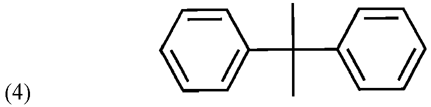 in Gegenwart von einem oder mehreren Lösungsmitteln, insbesondere von aprotischen Lösungsmitteln, umgesetzt werden.

Geeignete Gegenionen zu den Anionen der Formel (1) sind typischerweise kationische quartäre Ammoniumverbindungen, insbesondere Tetraalkylammonium, bevorzugt Tetrabutylammonium.

Ein bevorzugtes Verfahren ist daher dadurch gekennzeichnet, dass die Verbindungen der Formel (1) als Salze mit kationischen quartären Ammoniumverbindungen, insbesondere Tetraalkylammonium, bevorzugt Tetrabutylammonium als Gegenionen eingesetzt werden.

Bevorzugte Ausgangsverbindungen der Formel (1) sind solche ausgewählt aus der Reihe: 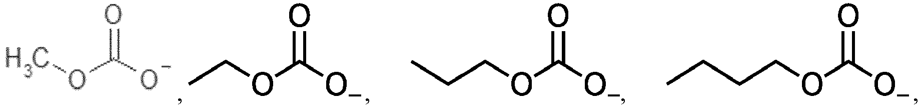 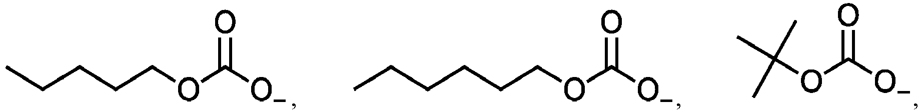 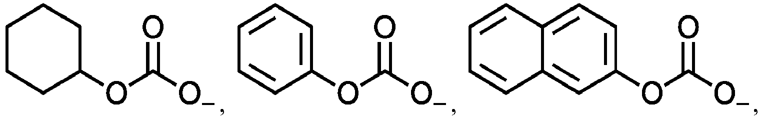 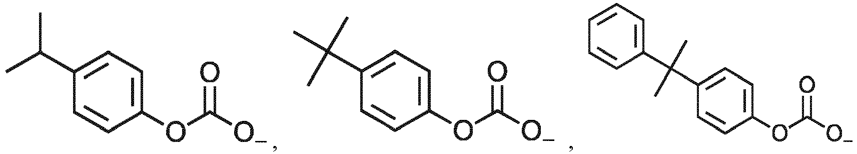 insbesondere bevorzugt solche der Reihe: 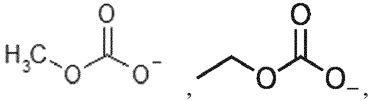 und

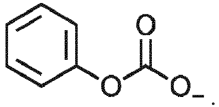

Besonders bevorzugte Ausgangsverbindungen der Formel (2) sind solche ausgewählt aus der Reihe: 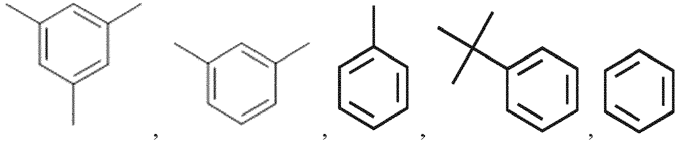 insbesondere bevorzugt der Formeln:

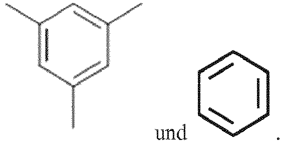

Besonders bevorzugt sind anodische Umsetzungen von 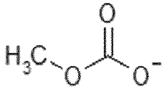 mit einer Verbindung aus der Reihe:

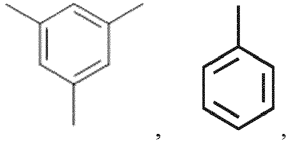

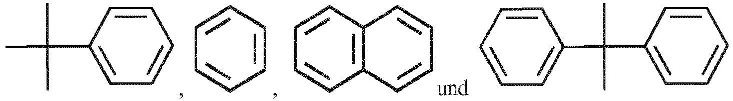

Besonders bevorzugt sind auch anodische Umsetzungen von 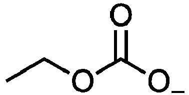 mit einer Verbindung der Reihe:

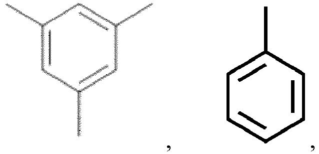

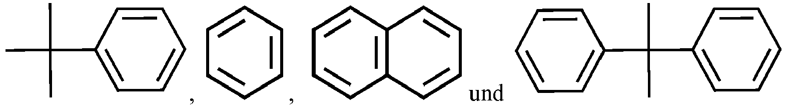

Besonders bevorzugt sind auch die Umsetzung von 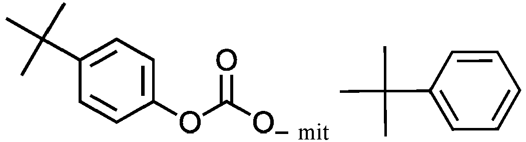 und die Umsetzung von

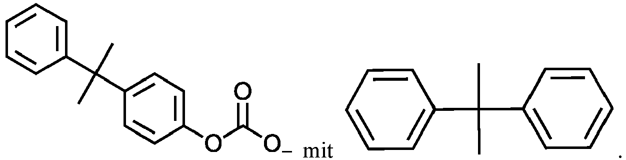

Ferner offenbart ist die Verwendung der aus dem neuen Verfahren erhältlichen Arylalkylcarbonate oder Diarylcarbonate zur Herstellung von Polycarbonaten.

Die Reaktionsgleichung des neuen elektrochemischen Herstellungsverfahrens sei am Beispiel der Umsetzung von 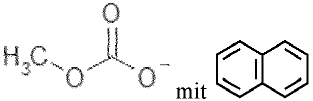 schematisch gezeigt:

Anodenreaktion:

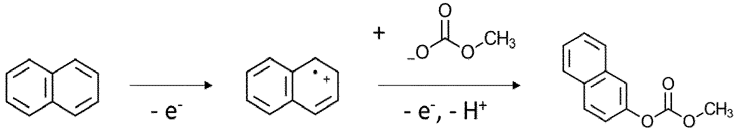

Als Kathodenreaktion dient beispielsweise die Wasserstoffentwicklung aus saurer Lösung:

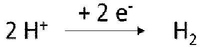

Die neue anodische elektrochemische Reaktion wird bevorzugt bei einer Stromdichte im Bereich von 0,5 - 100 mA/cm² und bei einer Temperatur im Bereich von 10 - 80°C durchgeführt.

Zur Durchführung des neuen Verfahrens können bevorzugt handelsübliche Bor-dotierte Diamant-elektroden eingesetzt werden, die als Anode geschaltet werden. Der Vorteil der Bor-dotierten Diamantelektrode besteht darin, dass sie unter anodischen Bedingungen chemisch sehr stabil ist, und insbesondere gegenüber radikalischen Zwischenstufen bei der elektrochemischen Umsetzung sehr beständig ist.

Diamantelektroden, die grundsätzlich für das neue Verfahren besonders geeignet sind, sind dadurch gekennzeichnet, dass eine elektrisch leitfähige Diamantschicht, die Bor-dotiert sein kann, auf einem geeigneten elektrisch leitenden Trägermaterial aufgebracht wird. Das meist angewandte Verfahren zur Herstellung solcher Elektroden ist die "Hot filament chemical vapor deposition"-Technik (HFCVD), um aktive und stabile Bor-dotierte Diamant-Elektroden herzustellen. Bei vermindertem Druck (Größenordnung 10 mbar) und hoher lokaler Temperatur (> 2000 °C), die durch Heizdrähte erzeugt wird, wird eine Kohlenstoffquelle (z.B. Methan) und Wasserstoff eingesetzt. Unter diesen Prozessbedingungen gebildete Wasserstoffradikale ermöglichen die Bildung von Methylradikalen, welche sich letztendlich als Diamant auf einem Trägermaterial abscheiden [M. Rüffer, "Diamond electrodes - properties, fabrication, applications," Vortrag auf ACHEMA 2015, Frankfurt am Main, 2015]. Für die elektrochemische Verwendung bedarf es leitfähiger Elektroden, weshalb die Diamantschicht im Herstellungsprozess mit Bor dotiert wird. Für die Bordotierung wird auf geringe Konzentrationen an Diborane, Trimethylboran, Bortrioxid oder Borate zurückgegriffen [L. Pan und D. Kanja, Diamond: Electronic Properties and Applications, Kluwer AcademicPublishers: Boston, 1995]. Gängig ist auch, einen zusätzlichen Wasserstoffgasstrom durch eine Methanol/Bortrioxid Lösung (mit definiertem C/B-Verhältnis) zu führen [E. Brillas und C. A. Martinez-Huitle, Synthetic Diamond Films: Preparation, Electrochemistry, Characterization and Applications, John Wiley & Sons, 2001]. In einer bevorzugten Ausführung der Erfindung wird die anodische Reaktion an einer Bor-dotierten Diamantelektrode als Anode durchgeführt, bei der die Bor-dotierte Diamantschicht auf verschiedenen Basismaterialien aufgebracht ist. So können unabhängig voneinander bevorzugt Titan, Silizium oder Niob als Trägermaterial für die Diamantschicht eingesetzt werden. Besonders bevorzugtes Trägermaterial ist Silizium. Auch andere Trägermaterialen, auf denen die Diamantschicht haftet und eine dichte Schicht bildet, können grundsätzlich eingesetzt werden.

Der elektrisch leitende Träger zur Herstellung der Bor-dotierten Diamantschicht kann grundsätzlich ein Netz, Vlies, Schaum, Gewebe, Geflecht oder Streckmetall sein. Bevorzugt wird ein Träger in Form eines Streckmetalls eingesetzt. Der Träger kann einlagig oder mehrlagig sein. Ein mehrlagiger Träger kann aus zwei oder mehreren übereinander angeordneten Netzen, Vliesen, Schäumen, Geweben, Geflechten oder Streckmetallen aufgebaut sein. Die Netze, Vliese, Schäume, Gewebe, Geflechte oder Streckmetalle können dabei unterschiedlich sein. Sie können z.B. unterschiedlich dick oder unterschiedlich porös sein oder eine unterschiedliche Maschenweite aufweisen. Zwei oder mehrere Netze, Vliese, Schäume, Gewebe, Geflechte oder Streckmetalle können z.B. durch Sintern oder Schweißen miteinander verbunden sein.

In einem bevorzugten Verfahren wird eine Bor-dotierte Diamantelektrode eingesetzt, die auf einem Träger basierend auf mindestens einem Material ausgewählt aus der Reihe: Tantal, Silizium und Niob, bevorzugt Silizium aufgebaut ist.

Eine weitere bevorzugte Ausführung des neuen Verfahrens ist dadurch gekennzeichnet, dass die Verbindungen der Formel 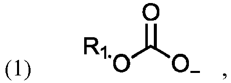 worin der Rest R₁ ein Alkylrest bedeutet, bevorzugt einen Rest aus der Reihe: C₁- bis C₆- Alkyl, oder Cycloalkyl, bevorzugt Cyclohexyl, oder ein Aryl-, Tert-butylphenyl-, Cumylphenyl- oder 4-Isopropylphenylrest, bevorzugt Naphthyl oder Phenyl bedeutet, ausgehend von dem entsprechenden Alkohol R₁OH durch eine der anodischen Reaktion vorgelagerte Umsetzung mit Kohlendioxid an einer Kathode elektrochemisch erzeugt werden.

Bevorzugt wird in der vorgelagerten kathodischen Reaktion als Alkohol R₁OH ein Alkohol aus der Reihe: Methanol, Ethanol, n-Propanol, n-Butanol, tert-Butanol, n-Pentanol, n-Hexanol, Cyclohexanol, Tert-Butylphenol, Cumylphenol, Napththol oder Phenol eingesetzt.

Die Reaktion zur kathodischen Bildung eines Monoalkylcarbonates kann grundsätzlich in analoger Weise wie beispielhaft in Tetrahedron Letters, 1997, 20. 3565-3568 beschrieben durchgeführt werden, wobei die dort beschriebene Zugabe eines Alkylierungsreagenzes wegfällt:
Hierbei wird beispielsweise eine Lösung von Et₄NClO₄ (0,1 mol/1) in Acetonitril mit CO₂ gesättigt. Unter kontinuierlicher CO₂-Zufuhr wird das CO₂ kathodisch an einer Kupfer- oder Platinelektrode umgesetzt bei einem Elektrodenpotential von -2,1 V (gegen Kalomelelektrode als Referenz) mit einer Ladung von 2 F pro Mol des später eingesetzten Alkohols bei Raumtemperatur in geteilter Zelle. Danach wird die Reaktionsmischung mit Stickstoff gespült und der entsprechende Alkohol zugegeben und 90 Minuten bei Raumtemperatur gerührt.

Die Kathodenreaktion zur Bildung eines Monoalkylcarbonates ist grundsätzlich in Novel Trends in Electroorganic Synthesis, [Papers presented at the International Symposium on Electroorganic Synthesis], 3rd, Kurashiki, Japan, Sept. 24-27, 1997 (1998), 193-196 beschrieben.

Es handelt sich hierbei um eine komplexe Abfolge von mehreren Reaktionen, die hier exemplarisch am Beispiel von Methanol gezeigt sei:

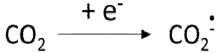

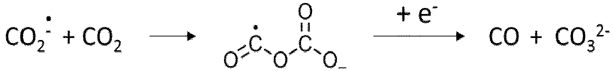

CO₃²⁻ + CH₃OH → HCO₃⁻ + CH₃O⁻

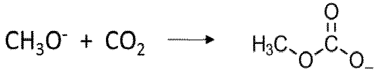

Die vorgelagerte kathodische Reaktion nach der bevorzugten Ausführung findet insbesondere in Acetonitril als Lösungsmittel statt, kann aber auch in anderen aprotischen Lösungsmitteln wie DMF, DMSO, 1,2-Dimethoxyethane oder N-methyl-2-pyrrolidon durchgeführt werden. Die vorgelagerte kathodische Reaktion kann insbesondere bei einer Stromdichte im Bereich von 2 - 100 mA/cm und einer Temperatur im Bereich von 10 - 80°C durchgeführt werden.

Offenbart ist auch die Verwendung der Arylalkylcarbonate und Diarylcarbonate erhalten aus dem erfindungsgemäßen Verfahren zur Herstellung von Polycarbonaten nach dem Schmelze-Umesterungsverfahren.

Die Umesterungsreaktion kann beispielhaft nach folgender Gleichung beschrieben werden:

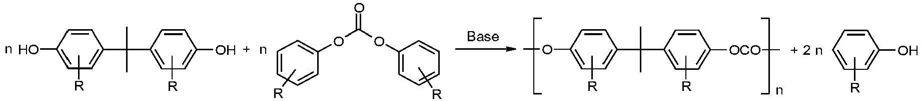

Im Folgenden werden bevorzugte weitere Einsatzstoffe und Verfahrensvarianten des an sich bekannten Schmelze-Umesterungsverfahrens zur Herstellung von Polycarbonaten genannt, die für die Umsetzung der aus dem erfindungsgemäßen Verfahren erhältlichen Arylalkylcarbonate und Diarylcarbonate Verwendung finden können.

Im Falle der Arylalkylcarbonate wird vor dem Schmelze-Umesterungsverfahrens noch eine grundsätzlich bekannte Umsetzung zu einem Diarylcarbonat vorgenommen. Dies wird beispielhaft in der EP2650278 bzw. EP1837328 beschrieben.

Die EP 2650278 beschreibt ein Verfahren zur Herstellung von Diarylcarbonaten für die Herstellung von Polycarbonat, wobei als Zwischenstufe ein Arylalkylcarbonat hergestellt wird. Eine Variante des erfindungsgemäßen Verfahrens liefert einen direkten Zugang zu Arylalkylcarbonaten, welche gemäß des in der EP 2650278 beschriebenen Verfahrens zur Herstellung von Diarylcarbonaten eingesetzt werden können. Es erfolgt hierbei eine Disproportionierungsreaktion, bei der Dialkylcarbonate und Diarylcarbonate entstehen. Die anfallenden Dialkylcarbonate können mit dem passenden Arylalkohol wieder in Arylalkylcarbonate umgesetzt werden, wobei Alkylalkohole freigesetzt werden. Solche Prozesse sind dem Fachmann bekannt und zum Beispiel ausführlich beschrieben in der EP1837328.

In dem Schmelze-Umesterungsverfahren zur Herstellung von Polycarbonaten erhaltene Polycarbonate können durch den Einsatz geringer Mengen Kettenabbrecher und Verzweiger bewusst und kontrolliert modifiziert werden. Geeignete Kettenabbrecher und Verzweiger sind literaturbekannt. Einige sind beispielsweise in der DE-A 38 33 953 beschrieben. Bevorzugt eingesetzte Kettenabbrecher sind Phenol oder Alkylphenole, insbesondere Phenol, p-tert.Butylphenol, iso-Octylphenol, Cumylphenol, deren Chlorkohlensäureester oder Säurechloride von Monocarbonsäuren bzw. Gemischen aus diesen Kettenabbrechern. Bevorzugte Kettenabbrecher sind Phenol, Cumyl-phenol, Isooctylphenol, para-tert.-Butylphenol.

Beispiele für als Verzweiger geeignete Verbindungen sind aromatische oder aliphatische Verbindungen mit mehr als drei, bevorzugt drei oder vier Hydroxygruppen. Besonders geeignete Beispiele mit drei oder mehr als drei phenolischen Hydroxylgruppen sind Phloroglucin, 4,6-Dimethyl-2,4,6-tri-(4-hydroxyphenyl)-hepten-2, 4,6-Dimethyl-2,4,6-tri-(4-hydroxyphenyl)-heptan, 1,3,5-Tri-(4-hydroxyphenyl)-benzol, 1,1,1 -Tri-(4-hydroxyphenyl)-ethan, Tri-(4-hydroxyphenyl)-phenyl-methan, 2,2-Bis-[4,4-bis-(4-hydroxyphenyl)-cyclohexyl]-propan, 2,4-Bis-(4-hydroxyphenyl-iso-propyl)-phenol, Tetra-(4-hydroxyphenyl)-methan.

Beispiele für sonstige als Verzweiger geeignete trifunktionelle Verbindungen sind 2,4-Dihydroxybenzoesäure, Trimesinsäure, Cyanurchlorid und 3,3-Bis-(3-methyl-4-hydroxyphenyl)-2-oxo-2,3-dihydroindol.

Besonders bevorzugte Verzweiger sind 3,3-Bis-(3-methyl-4-hydroxyphenyl)-2-oxo-2,3-dihydro-indol und 1,1,1-Tri-(4-hydroxyphenyl)-ethan.

Die gegebenenfalls mitzuverwendenden Verzweiger in einer Konentration von 0,05 bis 2 Mol-%, bezogen auf eingesetzte Bisphenole, können mit den Bisphenolen zusammen eingesetzt werden.

Es ist darauf zu achten, dass die Reaktionskomponenten für die Umesterung, die Bisphenole und die Diarylcarbonate, frei von Alkali- und Erdalkaliionen sind, wobei Mengen von kleiner 0,1 ppm an Alkali- und Erdalkaliionen toleriert werden können. Derart reine Bisphenole bzw. Diarylcarbonate sind erhältlich, indem man die Bisphenole bzw. Diarylcarbonate umkristallisiert, wäscht oder destilliert. Beim nachgeschalteten Umesterungsverfahren soll der Gehalt an Alkali- und Erdalkalimetallionen sowohl im Bisphenol als auch im Diarylcarbonat einen Wert von < 0,1 ppm betragen.

Die Umesterungsreaktion des Bisphenols und des Diarylcarbonats in der Schmelze wird bevorzugt in zwei Stufen durchgeführt. In der ersten Stufe findet das Aufschmelzen des Bisphenols und des Diarylcarbonats bei Temperaturen von 80 - 250°C, bevorzugt 100 - 230°C, besonders bevorzugt 120 - 190°C unter normalem Druck in 0 - 5 Stunden, bevorzugt 0,25 - 3 Stunden statt. Nach Zugabe des Katalysators wird durch Anlegen von Vakuum (bis zu 2 mm Hg) und Erhöhung der Temperatur (auf bis zu 260°C) durch Abdestillieren des Monophenols das Oligocarbonat aus dem Bisphenol und dem Diarylcarbonat hergestellt. Das so hergestellte Oligocarbonat hat eine mittlere Gewichtsmolmasse Mw (ermittelt durch Messung der rel. Lösungsviskosität in Dichlormethan oder in Mischungen gleicher Gewichtsmengen Phenol / o-Dichlorbenzol, geeicht durch Lichtstreuung) im Bereich von 2000 bis 18 000, bevorzugt von 4 000 bis 15 000. Hierbei wird die Hauptmenge an Monophenol (80 %) aus dem Prozess wiedergewonnen.

In der zweiten Stufe wird bei der Polykondensation durch weiteres Erhöhen der Temperatur auf 250 - 320°C, bevorzugt 270 - 295°C und einem Druck von < 2 mm Hg das Polycarbonat hergestellt. Hierbei wird der Rest an Monophenolen zurückgewonnen. Es können geringe Verluste an Monophenolen < 5 %, bevorzugt < 2 %, besonders bevorzugt < 1 % auftreten, verursacht durch Endruppen im Polycarbonat und Restmonophenol im Polycarbonat. Diese Verluste müssen durch entsprechende Mengen an Monophenol für die Herstellung des Diarylcarbonats ausgeglichen werden.

Geeignete Katalysatoren im Sinne des Schmelze-Umesterungsverfahrens sind alle anorganischen oder organischen basischen Verbindungen beispielsweise Lithium-, Natrium-, Kalium-, Cäsium-, Calcium-, Barium-, Magnesium-, -hydroxide, -carbonate, -halogenide, -phenolate, -diphenolate, - fluoride, -acetate, -phosphate, -hydrogenphosphate, -boranate, Stickstoff- und Phosphorbasen wie beispielsweise Tetramethylammoniumhydroxid, Tetramethylammoniumacetat, Tetramethylammoniumfluorid, Tetramethylammoniumtetraphenylboranat, Tetraphenylphosphoniumfluorid, Tetra-phenylphosphoniumtetraphenylboranat, Dimethyldiphenylammoniumhydoxid, Tetraethylammoniumhydroxid, DBU, DBN oder Guanidinsysteme wie beispielsweise das 1,5,7-Triazabi-cyclo-[4,4,0]-dec-5-en, 7-Phenyl-1,5,7-triazabicyclo-[4,4,0]-dec-5-en, 7-Methyl-1,5,7-triazabi-cyclo-[4,4,0]-dec-5-en, 7,7'-Hexylidendi-1,5,7-triazabi-cyclo-[4,4,0]-dec-5-en, 7,7'-Decylidendi-1,5,7-triazabicyclo-[4,4,0]-dec-5-en, 7,7'-Dodecyliden-di-1,5,7-tri-aza-bicyclo-[4,4,0]-dec-5-en oder Phosphazene wie bei-spielsweise die Phosphazen-Base P1-t-Oct = tert.-Octyl-imino-tris-(dimethylamino)-phosphoran, Phosphazen-Base P1-t-Butyl = tert.-Butyl-imino-tris-(dimethylamino)-phosphoran, BEMP = 2-tert.-Butylimino-2-diethylamino-1,3-dimethyl-perhydro-1,3,2-diaza-2-phos-phoran.

Diese Katalysatoren werden insbesondere in Mengen von 10<-2> bis 10<-8> Mol, bezogen auf 1 Mol Bisphenol, eingesetzt.

Die Katalysatoren können auch in Kombination (zwei oder mehrere) miteinander eingesetzt werden.

Beim Einsatz von Alkali-/Erdalkali-Metallkatalysatoren kann es vorteilhaft sein, die Alkali-/ Erdalkali-Metallkatalysatoren zu einem späteren Zeitpunkt (z.B. nach der Oligocarbonatsynthese bei der Polykondensation in der zweiten Stufe) zuzusetzen. Die Zugabe des Alkali-/Erdalkali-Metallkatalysators kann z.B. als Feststoff oder als Lösung in Wasser, Phenol, Oligocarbonat oder Polycarbonat erfolgen.

Die Mitverwendung von Alkali- bzw. Erdalkali-Metallkatalysatoren widerspricht nicht der vorstehend erwähnten Forderung nach Reinheit der Reaktionspartner.

Die Reaktion des Bisphenols und des Diarylcarbonats zum Polycarbonat kann beim Schmelze-Umesterungsverfahren diskontinuierlich oder bevorzugt kontinuierlich durchgeführt werden, beispielsweise in Rührkesseln, Dünnschichtverdampfern, Fallflmverdampfern, Rührkesselkaskaden, Extrudern, Knetern, einfachen Scheibenreaktoren und Hochviskosscheibenreaktoren.

Die aromatischen Polycarbonate erhältlich aus dem Schmelze-Umesterungsverfahren sollen insbesondere mittlere Gewichtsmolmassen Mw von 18000 bis 80000 vorzugsweise 19000 bis 50000 haben, ermittelt durch Messung der rel. Lösungsviskosität in Dichlormethan oder in Mischungen gleicher Gewichtsmengen Phenol / o-Dichlorbenzol, geeicht durch Lichtstreuung.

Es ist vorteilhaft, die aus dem Schmelze-Umesterungsverfahren des Bisphenols und des Diarylcarbonats zum Polycarbonat abgespaltenen und isolierten Monophenole vor dem Einsatz in die Diarylcarbonatsynthese aufzureinigen. Die Rohmonophenole, die beim Umesterungsprozeß isoliert werden, können je nach Umesterungsbedingungen und Destillationsbedingungen u.a. mit Diarylcarbonaten, dem Bisphenol, Salicylsäure, Isopropenylphenol, Phenoxybenzoesäurephenylester, Xanthon, dem Hydroxymonoarylcarbonat verunreinigt sein. Die Reinigung kann nach den üblichen Reinigungsverfahren, also z. B. Destillation oder Umkristallisation erfolgen. Die Reinheit der Monophenole liegt dann bei > 99 %, bevorzugt bei > 99,8 %, besonders bevorzugt bei > 99,95 %

Die Erfindung wird nachfolgend durch die Beispiele, welche jedoch keine Beschränkung der Erfindung darstellen, näher erläutert.

### Beispiele

### Verwendete Analysenmethoden:

### Gaschromatographie (GC/GCMS)

Gaschromatographische Untersuchungen (GC) erfolgten mit Hilfe des Gaschromatographen GC-2010 der Firma Shimadzu, Japan. Die Wechselwirkung der Probe mit der stationären Phase erfolgt in einer Quarzkapillarsäule ZB-5MSi der Firma Phenomenex, USA (Länge: 30 m; Innendurchmesser: 0,25 mm; Filmdicke der kovalent gebundenen stationären Phase: 0,25 µm; Vorsäule: 5 m, Trägergas: Wasserstoff; Injektortemperatur: 250 °C; Detektortemperatur: 310 °C; Programm: Methode "hart": 50 °C Starttemperatur für 1 min, Heizrate: 15 °C/min, 290 °C Endtemperatur für 8 min) gemessen. Gaschromatographische Massenspektren (GCMS) wurden mit Hilfe des Gaschromatographen GC-2010 kombiniert mit dem Massendetektor GCMS-QP2010 der Firma Shimadzu, Japan aufgenommen. Es wird an einer Quarzkapillarsäule ZB-5MSi der Firma Phenomenex, USA (Länge: 30 m; Innendurchmesser: 0,25 mm; Filmdicke der kovalent gebundenen stationären Phase: 0,25 µm; Vorsäule: 5 m, Trägergas: Helium; Injektortemperatur: 250 °C; Detektortemperatur: 300 °C; Programm: Methode "hart": 50 °C Starttemperatur für 1 min, Heizrate: 15 °C/min, 290 °C Endtemperatur für 8 min; GCMS: Temperatur der Ionenquelle: 200 °C) gemessen.

### NMR-Spektroskopie

Die NMR-spektroskopischen Untersuchungen wurden an Multikemresonanzspektrometem des Typs AV II 400 der Firma Bruker, Analytische Messtechnik, Karlsruhe, durchgeführt. Die verwendeten Lösungsmittel sind jeweils angegeben. Alle ¹H- und ¹³C-Spektren wurden gemäß dem Restgehalt an nicht deuteriertem Lösungsmittel nach der NMR Solvent Data Chart der Fa. Cambridge Isotopes Laboratories, USA, kalibriert. Die Zuordnung der ¹H- und ¹³C-Signale erfolgte falls notwendig mit Hilfe von H,H-COSY, H,C-HSQC und H,C-HMBC-Spektren. Die chemischen Verschiebungen sind als δ-Werte in ppm angegeben. Die Auswertung der NMR-Spektren erfolgte mit dem Programm MestReNova (Version: v10.0.1-14719). Für die Multiplizitäten der NMR-Signale wurden folgende Abkürzungen verwendet: s (Singulett), bs (breites Singulett), d (Dublett), t (Triplett), q (Quartett), m (Multiplett), sowie alle denkbaren Kombinationen dieser Abkürzungen. Alle Kopplungskonstanten J wurden mit der Anzahl der eingeschlossenen Bindungen in Hertz (Hz) angegeben.

### Beispiel 1

### Herstellung von Mesitylmethylcarbonat

### 1. Schritt: Herstellung von Tetrabutylammoniummethylcarbonat

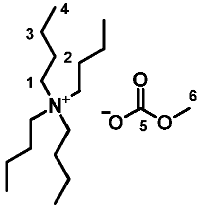

Die Synthese erfolgte in Anlehnung an die Vorschrift von Feroci et al. (J. Org. Chem. 2002, 67, 8287-8289). 29,3 g einer 21%igen methanolischen Tetrabutylammoniummethanolat-Lösung (6,16 g, 22,50 mmol) werden unter Argon in einem Einhalskolben vorgelegt und mit einem Gaseinleitungsrohr versehen. Durch zwei Gaswaschflaschen wird unter Rühren 4 Stunden Kohlendioxid-Gas eingeleitet. Nach beendeter Einleitung wird das Lösemittel unter vermindertem Druck entfernt. Der zähe Feststoff enthält noch Methanol, welches an einer Kugelrohrdestillationsvorrichtung im Hochvakuum bei Raumtemperatur entfernt wird. Der entstehende weiße Feststoff wird unter Hochvakuum getrocknet und unter Argon gelagert. Es werden 5,2 g (16,40 mmol) farbloser Feststoff erhalten. Die Ausbeute bezogen auf die Stoffmenge des eingesetzten Tetrabutylammoniummethanolats betrug 73%.
¹H-NMR (400 MHz, CDCl₃): δ (ppm) = 3.50 (s, 3H, H-6), 3.39-3.28 (m, 8H, H-1), 1.66-1.58 (m, 8H, H-2), 1.42 (h, J = 7.3 Hz, 8H, H-3), 0.98 (t, J = 7.3 Hz, 12H, H-4).
¹³C-NMR (101 MHz, CDCl₃): δ (ppm) = 158.27 (C-5), 58.72 (C-1), 52.12 (C-6), 23.98 (C-2), 19.67 (C-3), 13.61 (C-4).

### 2. Schritt: Herstellung von Mesitylmethylcarbonat bei 2 mA/cm²

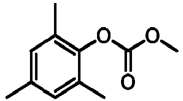

In einer ungeteilten 5 ml Teflonzelle werden 1 mmol des Mesitylens mit 3,5 mmol Tetrabutylammoniummethylcarbonat gelöst in 5 ml Acetonitril gegeben und an einer Bor-dotierten Diamant-Elektrode (DIACHEM®, 15 µm Diamantschicht auf Silizium-Trägermaterial, CONDIAS GmbH, Itzehoe, Deutschland)) anodisch umgesetzt. Als Gegenelektrode dient eine Platin-Kathode. Der Elektrodenabstand beträgt 0,5 cm. Die Elektrolyse erfolgt galvanostatisch mit einer Stromdichte von 2,0 mA/cm² und einer Ladungsmenge von 3,5 F bei Raumtemperatur (20°C) und Atmosphärendruck (1 atm). Nach Ende der Elektrolyse wird der Zellinhalt in einem Scheidetrichter zwischen 50 ml Wasser und 50 ml Ethylacetat separiert. Die organische Phase wird zweimal mit 50 ml Wasser gewaschen, mit Natriumsulfat getrocknet und das Lösungsmittel unter vermindertem Druck am Rotationsverdampfer entfernt.

Das erhaltene Rohprodukt wurde per präparativer HPLC an einer C18 Säule (KNAUER Wissenschaftliche Geräte GmbH, Deutschland, Eurospher II, Porengroße: 100 Å, Partikelgröße: 5 µm, Länge: 250 mm, Durchmesser: 30 mm) gereinigt. Methode: Flow 20 ml/min, Eluent ist Acetonitril (A) und Wasser 95%/Acetonitril 5%/Phosphorsäure (1 ml auf 1000 ml) (B), 0-40 min 15% A + 85% B, 40-120 min 100% A.

Es wurden 10 mg Produkt (0,05 mmol) als farblose Flüssigkeit erhalten. Die Ausbeute bezogen auf die Stoffmenge des eingesetzten Mesitylens betrug 5%.

Die Charakterisierung mit NMR ergab folgendes:
¹H-NMR (400 MHz, CDCl₃): δ (ppm) = 6.87 (s, 2H, H-3), 3.91 (s, 3H, H-8), 2.27 (s, 3H, H-6), 2.16 (s, 6H, H-5).
¹³C-NMR (101 MHz, CDCl₃): δ (ppm) = 153.76 (C-7), 146.15 (C-1), 135.59 (C-4), 129.66 (C-2), 129.32 (C-3), 55.49 (C-8), 20.75 (C-6), 15.93 (C-5).

MS (EI, GCMS): m/z 194 [M]⁺, 150 [M - 3 CH₃]⁺, 135 [M - CO₂CH₃]⁺, 119 [M-CO₃CH₃]⁺.

### 2. Schritt: Herstellung von Mesitylmethylcarbonat bei 3 mA/cm²

In einer ungeteilten 25 ml Glaszelle werden 2 mmol des Mesitylens mit 2 mmol Tetrabutylammoniummethylcarbonat gelöst in 20 ml Acetonitril gegeben und an einer Bor-dotierten Diamant-Elektrode (DIACHEM®, 15 µm Diamantschicht auf Silizium-Trägermaterial, CONDIAS GmbH, Itzehoe, Deutschland)) anodisch umgesetzt. Als Gegenelektrode dient eine Bor-dotierte Diamant- Kathode. Der Elektrodenabstand beträgt 0,4 cm. Die Elektrolyse erfolgt galvanostatisch mit einer Stromdichte von 3,0 mA/cm² und einer Ladungsmenge von 4,5 F bei Raumtemperatur (20°C) und Atmosphärendruck (1 atm). Nach Ende der Elektrolyse wird der Zellinhalt in einem Scheidetrichter zwischen 50 ml Wasser und 50 ml Ethylacetat separiert. Die organische Phase wird einmal mit 50 ml Wasser gewaschen, mit Magnesiumsulfat getrocknet und das Lösungsmittel unter vermindertem Druck am Rotationsverdampfer entfernt.

Das erhaltene Rohprodukt wurde mit einer Kugelrohrdestille (BÜCHI Labortechnik GmbH, Essen, Deutschland) gereinigt.

Es wurden 71 mg Produkt (0,37 mmol) als farblose Flüssigkeit erhalten. Die Ausbeute bezogen auf die Stoffmenge des eingesetzten Mesitylens betrug 18%.

Die Charakterisierung mit NMR ergab folgendes:
¹H-NMR (400 MHz, CDCl₃): δ (ppm) = 6.87 (s, 2H, H-3), 3.91 (s, 3H, H-8), 2.27 (s, 3H, H-6), 2.16 (s, 6H, H-5).
¹³C-NMR (101 MHz, CDCl₃): δ (ppm) = 153.76 (C-7), 146.15 (C-1), 135.59 (C-4), 129.66 (C-2), 129.32 (C-3), 55.49 (C-8), 20.75 (C-6), 15.93 (C-5).

MS (EI, GCMS): m/z 194 [M]⁺, 150 [M - 3 CH₃]⁺, 135 [M - CO₂CH₃]⁺, 119 [M-CO₃CH₃]⁺.

### Beispiel 2

### Herstellung von Naphthylmethylcarbonat

### 1. Schritt: Herstellung von Tetrabutylammoniummethylcarbonat

Die Herstellung von Tetrabutylammoniummethylcarbonat als Vorstufe erfolgt wie in Beispiel 1 unter Schritt 1 beschrieben.

### 2. Schritt: Herstellung von Naphthylmethylcarbonat

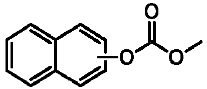

In einer ungeteilten 5 ml Teflonzelle werden 1,0 mmol des Naphthalins mit 3,5 mmol Tetrabutylammoniummethylcarbonat gelöst in 5 ml Acetonitril gegeben und an einer Bor-dotierten Diamant-Elektrode (DIACHEM®, 15 µm Diamantschicht auf Silizium-Trägermaterial, CONDIAS GmbH, Itzehoe, Deutschland) anodisch umgesetzt. Als Gegenelektrode dient eine Platin-Kathode. Der Elektrodenabstand beträgt 0,5 cm. Die Elektrolyse erfolgt galvanostatisch mit einer Stromdichte von 2,0 mA/cm² und einer Ladungsmenge von 2 F bei Raumtemperatur (20°C) und Atmosphärendruck (1 atm). Die Analyse der Reaktionsmischung erfolgte mittels GC-MS.

MS (EI, GCMS): m/z 208 [M]⁺, 135 [M - CO₂CH₂CH₃]⁺, 119 [M - CO₃CH₂CH₃]⁺ .

### Beispiel 3

### Herstellung von Mesitylethylcarbonat

### 1. Schritt: Herstellung von Tetrabutylammoniumethylcarbonat

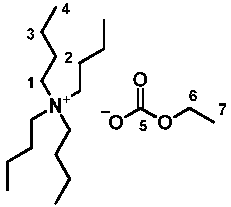

Die Synthese erfolgte in Anlehnung an die Vorschrift von Feroci et al. (J. Org. Chem. 2002, 67, 8287-8289). 20 ml einer 37,6%igen ethanolischen Tetrabutylammoniumethanolat-Lösung (7,52 g, 26,15 mmol) werden unter Argon in einem Einhalskolben vorgelegt und mit einem Gaseinleitungsrohr versehen. Durch zwei Gaswaschflaschen wird unter Rühren 4 Stunden Kohlendioxid-Gas eingeleitet. Nach beendeter Einleitung wird das Lösemittel unter vermindertem Druck entfernt. Der zähe Feststoff enthält noch Methanol, welches an einer Kugelrohrdestillationsvorrichtung im Hochvakuum bei Raumtemperatur entfernt wird. Der entstehende weiße Feststoff wird am Hochvakuum getrocknet und unter Argon gelagert. Es werden 3,7 g (11,12 mmol) farbloser Feststoff erhalten. Die Ausbeute bezogen auf die Stoffmenge des eingesetzten Tetrabutylammoniumethanolats betrug 43%.
¹H-NMR (400 MHz, CDCl₃): δ (ppm) = 3.90 (q, J = 7.1 Hz, 3H, H-6), 3.39-3.31 (m, 8H, H-1), 1.70-1.57 (m, 8H, H-2), 1.41 (h, J = 7.4 Hz, 8H, H-3), 1.15 (t, J = 7.1 Hz, 3H, H-7), 0.97 (t, J = 7.3 Hz, 12H, H-4).
¹³C-NMR (101 MHz, CDCl₃): δ (ppm) = 157.91 (C-5), 59.73 (C-7), 58.77 (C-1), 24.04 (C-2), 19.71 (C-3), 15.60 (C-7), 13.64 (C-4).

### 2. Schritt: Herstellung von Mesitylethylcarbonat

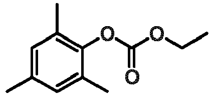

In einer ungeteilten 5 ml Teflonzelle werden 1,0 mmol des Mesitylens mit 3,5 mmol Tetrabutylammoniumethylcarbonat gelöst in 5 ml Acetonitril gegeben und an einer Bor-dotierten Diamant-Elektrode (DIACHEM®, 15 µm Diamantschicht auf Silizium-Trägermaterial, CONDIAS GmbH, Itzehoe, Deutschland) anodisch umgesetzt. Als Gegenelektrode dient eine Platin-Kathode. Der Elektrodenabstand beträgt 0,5 cm. Die Elektrolyse erfolgt galvanostatisch mit einer Stromdichte von 2,0 mA/cm² und einer Ladungsmenge von 2 F bei Raumtemperatur (20°C) und Atmosphärendruck (1 atm). Die Analyse der Reaktionsmischung erfolgte mittels GC-MS.

MS (EI, GCMS): m/z 208 [M]⁺, 135 [M - CO₂CH₂CH₃]⁺, 119 [M - CO₃CH₂CH₃]⁺ .

### Beispiel 4

### Herstellung von Xylylmethylcarbonat

### 1. Schritt: Herstellung von Tetrabutylammoniummethylcarbonat

Die Herstellung von Tetrabutylammoniummethylcarbonat als Vorstufe erfolgt wie in Beispiel 1 unter Schritt 1 beschrieben.

### 2_{.} Schritt: Herstellung von Xylylmethylcarbonat

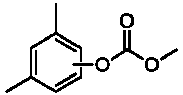

In einer ungeteilten 5 ml Teflonzelle werden 0,5 mmol des Xylols mit 0,5 mmol Tetrabutylammoniummethylcarbonat gelöst in 5 ml Acetonitril gegeben und an einer Bor-dotierten Diamant-Elektrode (DIACHEM®, 15 µm Diamantschicht auf Silizium-Trägermaterial, CONDIAS GmbH, Itzehoe, Deutschland) anodisch umgesetzt. Als Gegenelektrode dient eine Bor-dotierte Diamant-Kathode. Der Elektrodenabstand beträgt 0,5 cm. Die Elektrolyse erfolgt galvanostatisch mit einer Stromdichte von 2,0 mA/cm² und einer Ladungsmenge von 2 F bei Raumtemperatur (20°C) und Atmosphärendruck (1 atm). Die Analyse der Reaktionsmischung erfolgte mittels GC-MS.

MS (EI, GCMS): m/z 180 [M]⁺, 136 [M - 3 CH₃]⁺, 121 [M - CO₂CH₃]⁺ .

### Beispiel 5

### Herstellung von (1 ,3 -di(1, 1-dimethylethyl)-5-methylphenyl-methylcarbonat

### 1. Schritt: Herstellung von Tetrabutylammoniummethylcarbonat

Die Herstellung von Tetrabutylammoniummethylcarbonat als Vorstufe erfolgt wie in Beispiel 1 unter Schritt 1 beschrieben.

### 2. Schritt: Herstellung von (1,3-di(1,1-dimethylethyl)-5-methylphenyl)-methylcarbonat

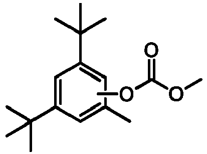

In einer ungeteilten 5 ml Teflonzelle werden 0,5 mmol des 5-Methyl-1,3-di(1,1-dimethylethyl)-benzols mit 0,5 mmol Tetrabutylammoniummethylcarbonat gelöst in 5 ml Acetonitril gegeben und an einer Bor-dotierten Diamant-Elektrode (DIACHEM®, 15 µm Diamantschicht auf Silizium-Trägermaterial, CONDIAS GmbH, Itzehoe, Deutschland) anodisch umgesetzt. Als Gegenelektrode dient eine Bor-dotierte Diamant- Kathode. Der Elektrodenabstand beträgt 0,5 cm. Die Elektrolyse erfolgt galvanostatisch mit einer Stromdichte von 2,0 mA/cm² und einer Ladungsmenge von 4,5 F bei Raumtemperatur (20°C) und Atmosphärendruck (1 atm). Die Analyse der Reaktionsmischung erfolgte mittels GC-MS.

MS (EI, GCMS): m/z 278 [M]⁺, 263 [M - CH₃]⁺, 219 [M - CO₂CH₃]⁺ .

### Beispiel 6

### Herstellung von (1,3,5-tri(1,1-dimethylethyl)-phenyl)-methylcarbonat

### 1. Schritt: Herstellung von Tetrabutylammoniummethylcarbonat

Die Herstellung von Tetrabutylammoniummethylcarbonat als Vorstufe erfolgt wie in Beispiel 1 unter Schritt 1 beschrieben.

### 2. Schritt: Herstellung von (1,3,5-tri(1,1-dimethylethyl)-phenyl)-methylcarbonat

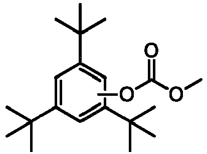

In einer ungeteilten 5 ml Teflonzelle werden 0,5 mmol des 1,3,5-Tri(1,1-dimethylethyl)-benzols mit 0,5 mmol Tetrabutylammoniummethylcarbonat gelöst in 5 ml Acetonitril gegeben und an einer Bor-dotierten Diamant-Elektrode (DIACHEM®, 15 µm Diamantschicht auf Silizium-Trägermaterial, CONDIAS GmbH, Itzehoe, Deutschland) anodisch umgesetzt. Als Gegenelektrode dient eine Bor-dotierte Diamant- Kathode. Der Elektrodenabstand beträgt 0,5 cm. Die Elektrolyse erfolgt galvanostatisch mit einer Stromdichte von 2,0 mA/cm² und einer Ladungsmenge von 4,5 F bei Raumtemperatur (20°C) und Atmosphärendruck (1 atm). Die Analyse der Reaktionsmischung erfolgte mittels GC-MS.

MS (EI, GCMS): m/z 320 [M]⁺, 305 [M - CH₃]⁺, 261 [M - CO₂CH₃]⁺ .

### Beispiel 7

### Herstellung von (1-(1,1-dimethylethyl)-3,5-dimethylphenyl)-methylcarbonat

### 1. Schritt: Herstellung von Tetrabutylammoniummethylcarbonat

Die Herstellung von Tetrabutylammoniummethylcarbonat als Vorstufe erfolgt wie in Beispiel 1 unter Schritt 1 beschrieben.

### 2. Schritt: Herstellung von (1-(1,1-dimethylethyl)-3,5-dimethylphenyl)-methylcarbonat

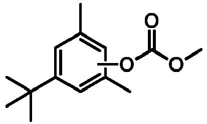

In einer ungeteilten 5 ml Teflonzelle werden 0,5 mmol des 3,5-Dimethyl-1-(1,1-dimethylethyl)-benzols mit 0,5 mmol Tetrabutylammoniummethylcarbonat gelöst in 5 ml Acetonitril gegeben und an einer Bor-dotierten Diamant-Elektrode (DIACHEM®, 15 µm Diamantschicht auf Silizium-Trägermaterial, CONDIAS GmbH, Itzehoe, Deutschland) anodisch umgesetzt. Als Gegenelektrode dient eine Bor-dotierte Diamant- Kathode. Der Elektrodenabstand beträgt 0,5 cm. Die Elektrolyse erfolgt galvanostatisch mit einer Stromdichte von 2,0 mA/cm² und einer Ladungsmenge von 4,5 F bei Raumtemperatur (20°C) und Atmosphärendruck (1 atm). Die Analyse der Reaktionsmischung erfolgte mittels GC-MS.

MS (EI, GCMS): m/z 236 [M]⁺, 221 [M -CH₃]⁻, 177 [M - CO₂CH₃]⁺, 161 [M - CO₃CH₃]⁺.

### Beispiel 8

### Herstellung von (1,3,5-tri(1-methylethyl)-phenyl)-methylcarbonat

### 1. Schritt: Herstellung von Tetrabutylammoniummethylcarbonat

Die Herstellung von Tetrabutylammoniummethylcarbonat als Vorstufe erfolgt wie in Beispiel 1 unter Schritt 1 beschrieben.

### 2. Schritt: Herstellung von (1,3,5-tri(1-methylethyl)-phenyl)-methylcarbonat

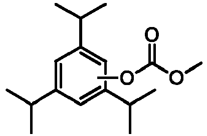

In einer ungeteilten 5 ml Teflonzelle werden 0,5 mmol des 1,3,5-Tri(1-methylethyl)-benzols mit 0,5 mmol Tetrabutylammoniummethylcarbonat gelöst in 5 ml Acetonitril gegeben und an einer Bor-dotierten Diamant-Elektrode (DIACHEM®, 15 µm Diamantschicht auf Silizium-Trägermaterial, CONDIAS GmbH, Itzehoe, Deutschland) anodisch umgesetzt. Als Gegenelektrode dient eine Bor-dotierte Diamant- Kathode. Der Elektrodenabstand beträgt 0,5 cm. Die Elektrolyse erfolgt galvanostatisch mit einer Stromdichte von 2,0 mA/cm² und einer Ladungsmenge von 2,5 F bei Raumtemperatur (20°C) und Atmosphärendruck (1 atm). Die Analyse der Reaktionsmischung erfolgte mittels GC-MS.

MS (EI, GCMS): m/z 278 [M]⁺, 263 [M - CH₃]⁺, 219 [M - CO₂CH₃]⁻, 203 [M - CO₃CH₃]⁻.

## Patentansprüche

1. Verfahren zur elektrochemischen Herstellung von Arylalkylcarbonaten und Diaryl carbonaten, **dadurch gekennzeichnet, dass** Verbindungen der Formel 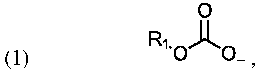 anodisch mit einer aromatischen Verbindung der Formel 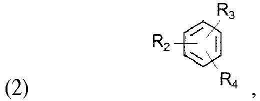 wobei der Rest R₁ einen Alkylrest bedeutet, bevorzugt einen Rest aus der Reihe: C₁- bis C₆- Alkyl, bevorzugt Methyl-, oder Ethyl- oder tert-Butyl, oder Cycloalkyl, bevorzugt Cyclohexyl oder einen Aryl-, Tert-butylphenyl-, Cumylphenyl- oder 4-Isopropylphenylrest, bevorzugt Naphthyl- oder Phenyl-, besonders bevorzugt einen Phenylrest bedeutet und die Reste R₂, R₃ und R₄ unabhängig voneinander Wasserstoff oder einen Alkylrest bedeuten, bevorzugt einen Rest aus der Reihe: C₁- bis C₆- Alkyl, iso-Propyl oder tert-Butyl oder Cycloalkyl, bevorzugt Cyclohexyl bedeuten,
oder mit einer aromatischen Verbindung der Formel 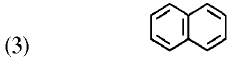 oder mit einer aromatischen Verbindung der Formel 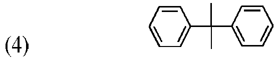 in Gegenwart von einem oder mehreren Lösungsmitteln, insbesondere von aprotischen Lösungsmitteln umgesetzt werden.

2. Verfahren nach Anspruch 1, **dadurch gekennzeichnet, dass** die anodische Reaktion an einer Bor-dotierten Diamantelektrode als Anode durchgeführt wird.

3. Verfahren nach einem der Ansprüche 1 oder 2, **dadurch gekennzeichnet, dass** als Lösungsmittel Acetonitril, oder eine Mischung von Acetonitril mit anderen Lösungsmittel insbesondere einem Lösungsmittel ausgewählt aus Dimethylformamid und Dimethylsulfoxid verwendet wird.

4. Verfahren nach einem der Ansprüche 1 bis 3, **dadurch gekennzeichnet, dass** der Rest R₁ in der Verbindung der Formel (1) ein Methylrest, Ethylrest oder ein Phenylrest, bevorzugt ein Methylrest oder Ethylrest ist.

5. Verfahren nach einem der Ansprüche 1 bis 4, **dadurch gekennzeichnet, dass** die Reste R₂, R₃ und R₄ in der Verbindung der Formel (2) unabhängig voneinander Wasserstoff oder Methyl, bevorzugt Wasserstoff bedeuten.

6. Verfahren nach einem der Ansprüche 1 bis 5, **dadurch gekennzeichnet, dass** die neue anodische elektrochemische Reaktion bei einer Stromdichte im Bereich von 0,5 - 100 mA/cm² und bei einer Temperatur im Bereich von 10 - 80°C durchgeführt wird.

7. Verfahren nach einem der Ansprüche 1 bis 6, **dadurch gekennzeichnet, dass** die Verbindungen der Formel (1) als Salze eingesetzt werden mit kationischen quartären Ammoniumverbindungen, insbesondere Tetraalkylammonium, bevorzugt Tetrabutylammonium als Gegenionen.

8. Verfahren nach einem der Ansprüche 1 bis 7, **dadurch gekennzeichnet, dass** die Verbindungen der Formel (1) 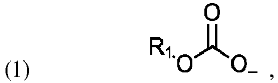 worin der Rest R₁ ein Alkylrest bedeutet, bevorzugt einen Rest aus der Reihe: C₁- bis C₆-Alkyl, oder Cycloalkyl, bevorzugt Cyclohexyl, oder ein Aryl-, Tert-butylphenyl-, Cumylphenyl- oder 4-Isopropylphenylrest, bevorzugt Naphthyl oder Phenyl bedeutet, ausgehend von dem entsprechenden Alkohol R₁OH durch eine der anodischen Reaktion vorgelagerte Umsetzung mit Kohlendioxid an einer Kathode elektrochemisch erzeugt werden.

9. Verfahren nach Anspruch 8, **dadurch gekennzeichnet, dass** in der vorgelagerten kathodischen Reaktion als Alkohol R₁OH ein Alkohol aus der Reihe: Methanol, Ethanol, n-Propanol, n-Butanol, tert-Butanol, n-Pentanol, n-Hexanol, Cyclohexanol, Tert-Butylphenol, Cumylphenol, Napththol oder Phenol eingesetzt wird.

## Claims

1. Process for the electrochemical preparation of aryl alkyl carbonates and diaryl carbonates, **characterized in that** compounds of the formula 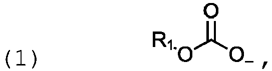 are anodically reacted with an aromatic compound of the formula 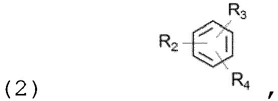 where the radical R₁ is an alkyl radical, preferably a radical selected from the group consisting of: C₁-C₆-alkyl, preferably methyl, ethyl or tert-butyl, or cycloalkyl, preferably cyclohexyl, or an aryl, tert-butylphenyl, cumylphenyl or 4-isopropylphenyl radical, preferably naphthyl or phenyl, particularly preferably a phenyl radical, and the radicals R₂, R₃ and R₄ are each, independently of one another, hydrogen or an alkyl radical, preferably a radical selected from the group consisting of: C₁-C₆-alkyl, isopropyl or tert-butyl or cycloalkyl, preferably cyclohexyl,
or with an aromatic compound of the formula 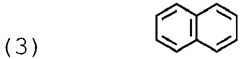 or with an aromatic compound of the formula 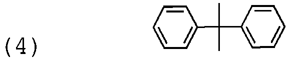 in the presence of one or more solvents, in particular of aprotic solvents.

2. Process according to Claim 1, **characterized in that** the anodic reaction is carried out at a boron-doped diamond electrode as anode.

3. Process according to either of Claims 1 and 2, **characterized in that** acetonitrile or a mixture of acetonitrile with other solvents, in particular a solvent selected from among dimethylformamide and dimethyl sulfoxide, is used as solvent.

4. Process according to any of Claims 1 to 3, **characterized in that** the radical R₁ in the compound of the formula (1) is a methyl radical, ethyl radical or a phenyl radical, preferably a methyl radical or ethyl radical.

5. Process according to any of Claims 1 to 4, **characterized in that** the radicals R₂, R₃ and R₄ in the compound of the formula (2) are each, independently of one another, hydrogen or methyl, preferably hydrogen.

6. Process according to any of Claims 1 to 5, **characterized in that** the novel anodic electrochemical reaction is carried out at a current density in the range 0.5-100 mA/cm² and at a temperature in the range 10-80°C.

7. Process according to any of Claims 1 to 6, **characterized in that** the compounds of the formula (1) are used as salts with cationic quaternary ammonium compounds, in particular tetraalkylammonium, preferably tetrabutylammonium, as counterions.

8. Process according to any of Claims 1 to 7, **characterized in that** the compounds of the formula (1) 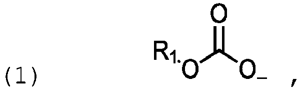 where the radical R₁ is an alkyl radical, preferably a radical selected from the group consisting of: C₁-C₆-alkyl, or cycloalkyl, preferably cyclohexyl, or an aryl, tert-butylphenyl, cumylphenyl or 4-isopropylphenyl radical, preferably naphthyl or phenyl, are electrochemically produced at a cathode from the corresponding alcohol R₁OH by means of a reaction with carbon dioxide preceding the anodic reaction.

9. Process according to Claim 8, **characterized in that** an alcohol selected from the group consisting of:
methanol, ethanol, n-propanol, n-butanol, tert-butanol, n-pentanol, n-hexanol, cyclohexanol, tert-butylphenol, cumylphenol, napththol or phenol is used as alcohol R₁OH in the preceding cathodic reaction.

## Revendications

1. Procédé pour la préparation électrochimique de carbonates d'aryle et d'alkyle et de carbonates de diaryle, **caractérisé en ce que** des composés de formule 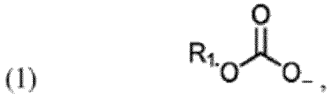 sont transformés de manière anodique avec un composé aromatique de formule 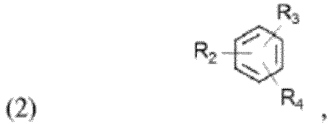 le radical R₁ signifiant un radical alkyle, préférablement un radical de la série : C₁₋₆-alkyle, préférablement méthyle, ou éthyle ou tert-butyle, ou cycloalkyle, préférablement cyclohexyle ou signifiant un radical aryle, tert-butylphényle, cumylphényle ou 4-isopropylphényle, préférablement naphtyle ou phényle, particulièrement préférablement un radical phényle et les radicaux R₂, R₃ et R₄ signifiant indépendamment les uns des autres hydrogène ou un radical alkyle, préférablement un radical de la série : C₁₋₆-alkyle, isopropyle ou tert-butyle ou cycloalkyle, préférablement signifiant cyclohexyle,
ou avec un composé aromatique de formule 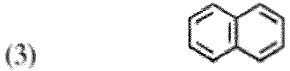 ou avec un composé aromatique de formule 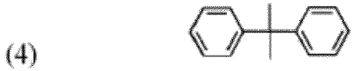 en présence d'un ou plusieurs solvants, en particulier de solvants aprotiques.

2. Procédé selon la revendication 1, **caractérisé en ce que** la réaction anodique est mise en œuvre au niveau d'une électrode de diamant dopé au bore en tant qu'anode.

3. Procédé selon l'une quelconque des revendications 1 et 2, **caractérisé en ce que** de l'acétonitrile, ou un mélange d'acétonitrile avec d'autres solvants en particulier un solvant choisi parmi le diméthylformamide et le diméthylsulfoxyde, est utilisé en tant que solvant.

4. Procédé selon l'une quelconque des revendications 1 à 3, **caractérisé en ce que** le radical R₁ du composé de formule (1) est un radical méthyle, un radical éthyle ou un radical phényle, préférablement un radical méthyle ou un radical éthyle.

5. Procédé selon l'une quelconque des revendications 1 à 4, **caractérisé en ce que** les radicaux R₂, R₃ et R₄ dans le composé de formule (2) signifient indépendamment les uns des autres hydrogène ou méthyle, préférablement hydrogène.

6. Procédé selon l'une quelconque des revendications 1 à 5, **caractérisé en ce que** la nouvelle réaction électrochimique anodique est mise en œuvre à une densité de courant dans la plage de 0,5 à 100 mA/cm² et à une température dans la plage de 10 à 80 °C.

7. Procédé selon l'une quelconque des revendications 1 à 6, **caractérisé en ce que** les composés de formule (1) sont utilisés en tant que sels avec des composés d'ammonium quaternaires cationiques, en particulier tétraalkylammonium, préférablement tétrabutylammonium en tant que contre-ions.

8. Procédé selon l'une quelconque des revendications 1 à 7, **caractérisé en ce que** les composés de formule (1) 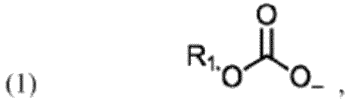 dans laquelle radical R₁ signifie un radical alkyle, préférablement un radical de la série C₁₋₆-alkyle, ou cycloalkyle, préférablement cyclohexyle, ou signifie un radical aryle, tert-butylphényle, cumylphényle ou 4-isopropylphényle, préférablement naphtyle ou phényle, sont générés de manière électrochimique au niveau d'une cathode à partir de l'alcool R₁OH correspondant par une transformation, située en amont de la réaction anodique, avec du dioxyde de carbone.

9. Procédé selon la revendication 8, **caractérisé en ce que** dans la réaction cathodique en amont, un alcool de la série : méthanol, éthanol, n-propanol, n-butanol, tert-butanol, n-pentanol, n-hexanol, cyclohexanol, tert-butylphénol, cumylphénol, naphtol ou phénol est utilisé en tant qu'alcool R₁OH.
